# EUROPEAN PATENT SPECIFICATION

(11) **EP 1 888 502 B1**
(45) Date of publication and mention of the grant of the patent: **13.07.2016**
(21) Application number: 06744885.2
(22) Date of filing: 09.05.2006
(51) Int. Cl.: C07C 67/293, C07C 69/007, C07D 307/79, C07D 317/54

(54) **CATALYTIC SCRIABINE REACTION**
KATALYTISCHE SCRIABINE-REAKTION
REACTION CATALYTIQUE DE SCRIABINE

(30) Priority: 11.05.2005 WO PCT/IB2005/001310
(43) Date of publication of application: 20.02.2008
(73) Proprietor: FIRMENICH SA, 1211 Geneva 8 (CH)
(72) Inventor: SNOWDEN, Roger, F-74580 Viry (FR); BIRKBECK, Anthony, CH-1205 Geneva (CH); WOMACK, Gary, Hopewell, New Jersey 08648 (US)
(74) Representative: Carina, Riccardo Filippo
(86) International application number: PCT/IB2006/051451
(87) International publication number: WO 2006/120639

(56) References cited:
- WO-A-2004/054997
- JP-A- 61 161 241
- US-A- 3 548 006
- CHAKRABORTI, ASIT K. ET AL: "Copper(II) tetrafluoroborate-catalyzed formation of aldehyde-1,1-diacetates" SYNTHESIS, no. 6, 2004, pages 831-833, XP002405203
- DATABASE CAPLUS [Online] CHEMICAL ABSTRACTS SERVICE, COLUMBUS, OHIO, US; INOUE, KAZUTAKA ET AL: "Alkenylidene diacetates" XP002405209 retrieved from STN Database accession no. 1987:119296 & JP 61 151152 A2 (MITSUBISHI RAYON CO., LTD., JAPAN) 9 July 1986 (1986-07-09)
- DATABASE CAPLUS [Online] CHEMICAL ABSTRACTS SERVICE, COLUMBUS, OHIO, US; RANU, BRINDABAN C. ET AL: "Zinc tetrafluoroborate-catalyzed efficient conversion of aldehydes to geminal diacetates and cyanoacetates" XP002405208 retrieved from STN Database accession no. 2003:301861 & CHEMISTRY LETTERS, vol. 32, no. 4, 2003, pages 366-367,
- LI, YI-QUN: "A rapid and convenient synthesis of 1,1-diacetates from aldehydes and acetic anhydride catalyzed by PVC-FeCl3 catalyst" SYNTHETIC COMMUNICATIONS, vol. 30, no. 21, 2000, pages 3913-3917, XP001247839
- SCRIABINE I: "no 185 - Nouveau procede de preparation des aldehydes dihydrocinnamiques" BULLETIN DE LA SOCIETE CHIMIQUE DE FRANCE. MEMOIRES, MASSON, PARIS, FR, 1961, pages 1194-1198, XP002977493 ISSN: 0366-3132 cited in the application

## Description

### Technical field

The present invention relates to the field of organic synthesis. More particularly it provides a process for making aromatic non-conjugated enol esters or enol ethers from an aromatic compound or moiety and a protected enal compound or moiety, such as an acetal or an acylal. The reaction is promoted by the use of some metal derivatives.

### Prior art

The Scriabine reaction consists of the reaction between an aromatic compound and an enal or the corresponding acylal (see I. Scriabine in Bull. Soc. Chem.Fr., 1961, 1194). This reaction provides an access to the formation of dihydrocinnamic aldehyde derivatives. To the best of our knowledge, all the methods and examples reported in the literature concerning this reaction are at least steochiometric and/or very strong acids (Al salt or in TiCl₄), see JP61161241. For instance one may cite Aguillar et al. in Synthetic Comm. 2004, 2719. The only exception concerns EP 1574509, but in said document the are used as substrate only highly electron rich substrates.

It is therefore highly desirable to access such dihydrocinnamic aldehyde derivatives by using a catalyzed reaction, and, if possible, catalysts which are more environmentally friendly.

### Description of the invention

In order to solve the aforementioned problems, the present invention provides a process for making a compound of the formula 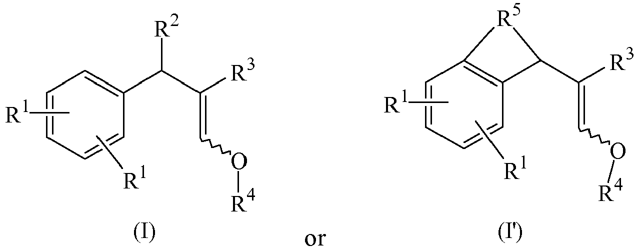 wherein the wavy line indicates that the double bond can be in a configuration E or Z or a mixture thereof;
each R¹ , when taken separately, one represents a hydrogen atom and the other a C₁-C₄ alkyl group; or the two R¹ , when taken together, represent a C₃-C₅ alkanediyl or alkenediyl group optionally substituted;
R² or R³ represents, taken separately, a hydrogen atom or a C₁-C₆ alkyl group; R² and R³, taken together, may represent a C₃-C₁₀ alkanediyl or alkenediyl group optionally substituted;
R⁴ represents a C₁-C₇ alkyl or fluorinated alkyl group, a C₇-C₁₀ alkylaromatic optionally substituted, a C₁-C₇ acyl group, or a -COCOOH or -COCH₂COOH group; and R⁵ represents a C₂-C₉ alkanediyl or alkenediyl group optionally substituted; comprising the coupling of a compound of formula (II) with a compound of formula (III) 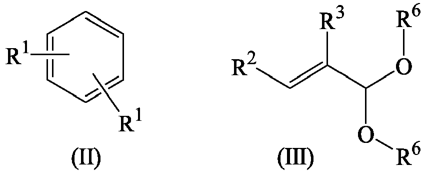 wherein **R¹** to R³ have the meaning indicated in formula (I) and each R⁶, taken separately, represents a C₁-C₇ alkyl or fluorinated alkyl group, a C₇-C₁₀ alkylaromatic optionally substituted, a C₁-C₇ acyl group, or the R⁶, taken together, represent a COCO or COCH₂CO group;
or, respectively, the cyclisation of a compound of formula 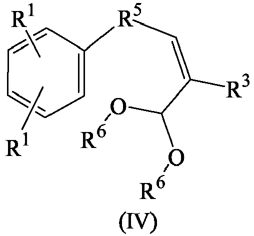 wherein R¹ and R³ have the meaning indicated in formula (I), R⁶ has the meaning indicated in formula (III), and R⁵ has the meaning indicated in formula (I'); said processes being characterized in that it is carried out in the presence of a catalytic amount of one catalyst selected from the group consisting of
- a salt of formula MXₙ, M representing a transition metal selected from the group consisting of Fe, Co, Ni, Cu and Zn, X representing a mono-anion and n is an integer from 1 to 3; and
- a boron compound of formula BY₃, wherein Y represents a fluoride or a phenyl group optionally substituted, and anyone of its adducts with a C₂-C₁₀ ether or a C₁-C₈ carboxylic acid.

Possible substituents of R¹ to R⁶ are one, two or three halogen atoms or OR^{a}, NR^{a}₂ or R^{a} groups, in which R^{a} is a hydrogen atom or a C₁ to C₁₀ cyclic, linear or branched alkyl or alkenyl group, preferably a C₁ to C₄ linear or branched alkyl or alkenyl group.

Possible substituents of Y are one to five groups such as halide atoms or methyl or CF₃ groups.

It is also understood that, when R¹ are not hydrogen atoms, the compound of formula (I), or (I'), can be in the form of a mixture of isomers. For example, if the compound of formula (II) is methyl-benzene, then the compound (I) obtained can be in the form of a mixture of the ortho, or meta, and para isomers.

According to a first embodiment of the invention, the invention provides a process for making a compound of formula (I) or (I') wherein R⁴ represents a C₁-C₇ alkyl group, a benzyl group optionally substituted or a C₁-C₇ acyl group.

According to a further embodiment, R² or R³ may represent, taken separately, a hydrogen atom or a C₁-C₄ alkyl group; R² and R³, taken together, may represent a C₃, C₄ or C₁₀ alkanediyl or alkenediyl group optionally substituted.

Furthermore, R⁵ may also represent a C₂-C₃ alkanediyl or alkenediyl group optionally substituted.

It is understood that in such embodiment the starting material are the corresponding compounds of formula (II) and (III), or the corresponding compound of formula (IV).

According to a further embodiment of the present invention the invention provides a process for making a compound of formula (I) by the reaction of a compound of formula (II) with a compound of formula (III).

As non limiting examples of compound of formula (II) one may cite the following: benzene optionally substituted by one or two C₁-C₄ alkyl groups, 1,3-benzodioxole or indane optionally substituted by one or two C₁-C₄ alkyl groups, and in particular 1,1-dimethyl indane.

As non limiting examples of compound of formula (III) one may cite the following: acrolein diethyl acetal, acrolein diacetate, methacrolein diacetate, crotonaldehyde diacetate, tiglyl diacetate, cyclohexenyl carbaldehyde diacetate.

As mentioned above the invention process is carried out in the presence of one catalyst which is a salt of formula MXₙ or a compound of formula BY₃ and adducts thereof. Said catalyst can be in the anhydrous form or also in the hydrate form, except for those acids which are unstable in the presence of water. However the anhydrous form is preferred.

Furthermore, according to a particular embodiment of the invention the use of one compound of formula MXₙ as catalysts is also preferred.

According to a particular embodiment of the invention, the catalyst is selected from the group consisting of BY₃ and adducts thereof.

According to a particular embodiment of the invention, the catalyst is selected from the group consisting of BY₃ and its adducts above mentioned.

As mentioned above BY₃ can be used alone or in the form of one of its adducts with an ether or a carboxylic acid. Specific examples are the adducts of BF₃ with Et₂O, Bu₂O or AcOH.

According to another embodiment of the invention, X is a mono-anion selected from the group consisting of acetylacetonate optionally substituted, Cl⁻, Br⁻, C₁₋₉ carboxylate, a C₁₋₁₀ sulphonate, ClO₄⁻, BF₄⁻, PF₆⁻ , SbCl₆⁻, AsCl₆⁻, SbF₆⁻, AsF₆⁻, BR⁷₄⁻, wherein R⁷ is a phenyl group optionally substituted by one to five groups such as halide atoms or methyl or CF₃ groups, or a R⁸SO₃⁻, wherein R⁸ is a chlorine or fluoride atom. In particular X can be selected from the group consisting of Cl⁻, Br⁻ and trifluoromethylsulfonate.

According to another embodiment of the invention, Y is F or C₆H₅.

According to a further embodiment of the invention, the catalyst is BF₃ and its adducts with AcOH.

The catalyst can be added to the reaction medium in a large range of concentrations. As non-limiting examples, one can cite catalyst concentrations ranging from 0.001 to 0.30 molar equivalents, relative to the molar amount of the starting compound (II) or (IV). Preferably, the catalyst concentrations will be comprised between 0.005 and 0.15 molar equivalents. It goes without saying that the optimum concentration of catalyst will depend on the nature of the catalyst and on the desired reaction time.

One can also cite catalyst concentrations ranging from 0.1 to 0.30 molar equivalents, relative to the molar amount of the starting compound (III). Preferably, the catalyst concentrations will be comprised between 0.01 and 0.10 molar equivalents. It goes without saying that the optimum concentration of catalyst will depend on the nature of the catalyst and on the desired reaction time.

It is useful here to mention that by "catalytic amount" we mean here any amount which allow the formation of the desired compound with a molar yield which exceeds the molar equivalents of catalyst added to the reaction mixture.

The temperature at which the invention's process can be carried out is typically between 0°C and 180°C, more preferably in the range of between 15°C and 100°C. Of course, a person skilled in the art is also able to select the preferred temperature as a function of the melting and boiling point of the starting and final products. Of course, a person skilled in the art is also able to select the preferred temperature as a function of the melting and boiling point of the starting and final products as well as of the solvent.

The process of the invention can be carried out in the presence or in the absence of solvent. As a person skilled in the art can anticipate, the presence of a solvent is mandatory only in the case in which the starting compound is a solid compound under the reaction conditions.

However, according to a preferred embodiment of the invention, and independently of the physical state of the starting compound, the process is advantageously carried out in the presence of a solvent. Preferably, said solvent is anhydrous or does not contain more than 1 % w/w water.

Non-limiting examples of such a solvent are C₄-C₈ ethers, C₃-C₆ esters, C₃-C₆ amides, C₆-C₉ aromatic solvents, C₅-C₇ linear or branched or cyclic hydrocarbons, C₁-C₂ chlorinated solvents and mixtures thereof.

Furthermore, the reaction can also be carried out in the presence of a solvent belonging to the family of carboxylic anhydride of formula R⁹C(O)O(O)CR⁹, R⁹ representing a C₁-C₇ alkyl group, a C₇-C₁₀ alkylaromatic optionally substituted, a C₁-C₇ acyl group, optionally containing the corresponding carboxylic acid R⁹COOH. The optional substituents being the same as for R₆.

The compound of formula (III) or (IV) can be made and isolated according to any prior art method. Alternatively, compound (III) or (IV) can be also generated *in situ,* i.e. in the reaction medium just before its use, according to any know prior art method.

In particular, preferably the compound of formula (III) or (IV) is made or generated by a method using the corresponding enal as starting material.

Therefore, another object of the present invention is an invention's process, as defined above, further comprising the step of generating *in situ* the compound of formula (III) or (IV) starting from the corresponding enal of formula (V) or (V') respectively 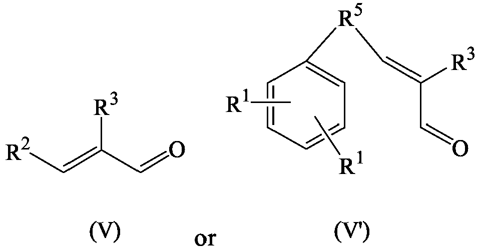 wherein R¹, R², R³ and R⁵ have the same meaning indicated above.

A process comprising the *in situ* generation of the compound of formula (III) or (I') is particularly useful when said compound (III) or (I') is an acetal or an acylal, the latter being a geminal dicarboxylate.

Now, when the compound of formula (II) is an acylal, we have also noticed that the catalysts that are able to promote the cyclisation of the acylal are also useful to promote the conversion of the enal into the corresponding acylal.

Therefore, another object of the present invention, and in fact a particular embodiment of the above-mentioned process, is a process for making a compound of formula (I) or (I'), as defined above, comprising the step of reacting, in the presence of a catalyst as defined above, an enal of formula (V) or (V'), as defined above, with a carboxylic anhydride of formula R⁹C(O)O(O)CR⁹, wherein R⁹ has the meaning indicated above.

The invention will now be described in further detail by way of the following examples, wherein the abbreviations have the usual meaning in the art, the temperatures are indicated in degrees centigrade (°C). The NMR spectral data were recorded in CDCl₃ at 400MHz or 100MHz for ¹H or ¹³C, respectively, the chemical displacements δ are indicated in ppm with respect to TMS as standard, and the coupling constants J are expressed in Hz. All the abbreviations have the usual meaning in the art. Each NMR spectra is provided in respect of the mayor isomer obtained, unless differently specified.

### Example 1

### Reaction between acrolein diacetate and 2-methyl indane

A solution of FeCl₃.6H₂O in acetic acid (1.0M, 1.0 ml, 1 mmol) was added slowly dropwise to acetic anhydride (20.4 g, 200 mmol) at 5°C. The solution was allowed to warm to room temperature. A solution of acrolein (5.6 g, 100 mmol), in 2-methyl indane (20.0 g, 151 mmol) and dichloromethane (15 g) was added slowly dropwise to the anhydride solution, maintaining the temperature at about 15°C. The mixture was stirred at 20°C for 4 hours then diluted with ethyl acetate (150 ml), and a saturated aqueous NaHCO₃ solution (50 ml) was added slowly dropwise. The aqueous phase was re-extracted with ethyl acetate (150 ml). The combined organic phases were washed with saturated aqueous NaHCO₃ solution (100 ml), brine (100 ml), dried over MgSO₄ and the solvents removed in vacuo. Further purification by KugelRohr distillation at 150°C (2.9 x 10⁻¹ mbar) gave the desired enol acetate as a mixture of isomers (7.2 g, 30%).
¹H-NMR: 1.13 (d, J 6.2, 3H), 2.11 (s, 3H), 2.43-2.59 (m, 3H), 2.98-3.06 (m, 2H), 3.29 (d, J 8.2, 2H), 5.53-5.60 (m, 1H), 6.95 (d, J 7.2, 1H), 7.02 (s, 1H), 7.10 (d, J 7.7, 1H), 7.18 (dt, J 13.8,1, 1H).
¹³C-NMR: 20.7 (q), 20.9 (q), 33.5 (t), 34.65 (d), 40.7 (t), 41.0 (t), 114.2 (d), 124.4 (d), 126.0 (d), 136.0 (d), 137.5 (s), 141.8 (s), 144.2 (s), 168.2 (s).

### Example 2

### Reaction between acrolein diacetate and 2-methyl indane

A suspension of 2-methyl indane (2.6 g, 20 mmol) and acrolein diacetate (1.6 g, 10 mmol) and zinc bromide (0.25 g, 1 mmol) was stirred for 24 hours at ambient temperature. The reaction medium was then diluted with ethyl acetate (50 ml), and a saturated aqueous NaHCO₃ solution (50 ml) was added slowly dropwise. The aqueous phase was re-extracted with ethyl acetate (50 ml). The combined organic extracts were washed with saturated aqueous NaHCO₃ solution (50 ml), brine (50 ml), dried over MgSO₄, filtered and the solvents removed in vacuo. Further purification of the residue by KugelRohr distillation 180°C (8.0 x 10⁻¹ mbar) gave the enol acetate as a mixture of isomers (0.65 g, 30%) identical to that prepared above.

### (Reference example)

### Example 3

### Reaction between 2,2 dimethyl dihydrobenzofuran and acrolein diacetate

Zinc bromide (50 mg, 0.2 mmol) was suspended in a solution of acrolein diacetate (1.6 g, 10 mmol), 2,2 dimethyl dihydrobenzofuran (1.5 g, 10 mmol), in dichloromethane (5 g) and the stirred at ambient temperature for 24 hours. The reaction medium was then diluted with ethyl acetate (25 ml) and the saturated aqueous NaHCO₃ solution (20 ml) was added slowly dropwise. The aqueous phase was re-extracted with ethyl acetate (25 ml). The combined organic phase was washed with saturated aqueous NaHCO₃ solution (25 ml), brine (25 ml), then dried over MgSO₄, filtered and the solvents removed in vacuo. Further purification by KugelRohr distillation 160°C (3.3 x 10⁻¹ mbar) gave the desired enol acetate as a mixture of isomers (0.9 g, 37%).
¹H-NMR: 1.45 (s, 6H), 2.11 (s, 3H), 2.97 (s, 2H), 3.25 (d, J 7.7, 2H), 5.55 (dt, 12.3, 7.7, 1H), 6.64 (d, 8.2, 1H), 6.91 (d, J 8.2, 1H), 6.96 (s, 1H), 7.16 (d, 12.3, 1H).
¹³C-NMR: 20.7 (q), 28.3 (q), 33.0 (t), 42.9 (t), 86.6 (s), 109.2 (d), 114.5 (d), 125.1 (d), 127.4 (s), 127.8 (d), 131.2 (s), 136.0 (d), 157.5 (s), 168.2 (s).

### Example 4

### Reaction between 2-methyl indane and methacrolein diacetate

BF₃ acetic acid complex (0.2 g, 1 mmol) was added to a stirred solution of 2-methyl indane (13.2 g, 100 mmol) and methacrolein diacetate (8.7 g, 50 mmol) heated at 60°C. The mixture was stirred at 60°C for one hour, then cooled and diluted with ethyl acetate (50 ml), and saturated aqueous NaHCO₃ solution (50 ml) was added slowly dropwise. The aqueous phase was re-extracted with ethyl acetate (50 ml), the combined organic phase was washed with NaHCO₃ (100 ml), dried over MgSO₄, filtered and the solvents removed *in vacuo.* The residue was further purified by KugelRohr distillation 160°C (6.0 x 10⁻¹ mbar) to give the enol acetate as a mixture of isomers (2.3 g, 19%).
¹H-NMR: (major isomer only) 1.13 (d, J 6.7, 3H), 1.60 (d, J 1.5, 3H), 2.14 (s, 3H), 2.44-2.59 (m, 3H), 2.97-3.05 (m, 2H), 3.22 (s, 2H), 6.93 (d, J 6.7, 1H), 6.99 (s, 1H), 7.05 (d, J 1.5, 1H), 7.08 (d, J 7.2, 1H).
¹³C-NMR: 13.6 (q), 20.8 (q), 20.7 (q), 34.7 (d), 40.2 (t), 40.8 (t), 41.1 (t), 121.6 (s), 124.2 (d), 124.8 (d), 126.6 (d), 131.1 (d), 136.8 (s), 141.8 (s), 144.1 (s), 168.3 (s).

### Example 5

### Reaction between an aromatic and acroleine diacetate

### A) General procedure

A solution of FeCl₃.6H₂O in acetic acid (1.0M, 2-3 ml, 2-3 mmol, 5-10% mol) was added to a stirred solution of the indane derivative (35 mmol) acetic anhydride (2 g) and acrolein diacetate (6.5 g, 41 mmol) cooled to 0°C. Stirred for a further 60 minutes at ambient temperature, diluted with ethyl acetate (50 ml), and added saturated sodium bicarbonate (25 ml) slowly dropwise. The aqueous phase was re-extracted with ethyl acetate (50 ml), the combined organic phase was washed with bicarbonate (50 ml) then brine (50 ml), dried over magnesium sulfate, filtered and the solvents *removed in vacuo.* The crude product was purified by Kugelrohr distillation, firstly under moderate vacuum (5-10 mbar) to recover the indane, then under high vacuum (1.0-4.0 x 10⁻¹ mbar).

### 2-ethyl indane

### yield (18%), b.p 160°C at 2.8 x 10⁻¹ mbar

¹H-NMR: 0.96 (t, J 7.2, 3H), 1.50 (quintet, J 7.2, 2H), 2.10 (s, 3H), 2.34 (septet, J 7.2, 2H), 2.60-2.46 (m, 2H), 2.95-3.05 (m, 2H), 3.28 (d, J 7.7, 2H), 5.51-5.60 (m, 1H), 6.94 (d, J 7.7, 1H), 7.01 (s, 1H), 7.09 (d, J 7.7, 1H) 7.17 (dt, J 12.3, 1.5, 1H)
¹³C-NMR: 12.8 (q), 20.7 (q), 28.7 (t), 33.5 (t), 38.6 (t), 38.9 (t), 42.2 (d), 114.3 (d), 124.4 (d), 126.1 (d), 136.1 (d), 137.6 (s), 141.8 (s), 144.2 (s), 168.2.

### 2-propyl indane

### yield 1.4 g, 18%, b.p 150°C at 2.5 x 10⁻¹ mbar)

¹H-NMR: 0.88-0.95 (m, 3H), 1.36-1.51 (m, 4H), 2.14 (s, 3H), 2.41-2.59 (m, 2H), 2.95-3.05 (m, 2H), 3.28 (d, J 7.7, 2H), 5.56 (dt, J 12.8,7.2, 1H), 6.94 (d, J 7.7, 1H), 7.01 (s, 1H), 7.08-7.19 (m, 2H).
¹³C-NMR: 14.3 (q), 20.7 (q), 21.5 (t), 33.5 (t), 38.1 (t), 38.9 (t), 40.20 (d), 114.3 (d), 124.4 (d), 126.0 (d), 126.1 (d), 136.1 (d), 137.5 (s), 141.8 (s), 143.7 (s), 144.2 (s), 168.2 (s).

### 2,2 dimethyl indane

purified by column chromatography over silica (200 ml) with ether:pentane as eluant (1:19 then 1:9) gave the desired enol acetate (1.1 g, 14%).
¹H-NMR: 1.13 (s, 6H), 2.11 (s, 3H), 2.65-2.73 (m, 4H), 3.28 (d, J 7.7, 2H), 5.50-5.60 (m, 1H), 6.94 (d, J 7.7, 1H), 6.98 (s, 1H), 7.07 (d, J 7.7, 1 H), 7.18 (dt, J 10.8, 1.5, 1 H).
¹³C-NMR: 20.8 (q), 28.8 (q), 33.5 (t), 40.2 (s), 47.4 (t), 47.7 (t), 114.3 (d), 124.7 (d), 126.0 (d), 136.1 (d), 137.5 (s), 141.6 (s), 144.0 (s), 168.2 (s).

### cis trans 1,2 dimethyl indane

### yield 1.7 g, 28%, b.p 150°C at 4.5 x 10⁻¹ mbar.

¹H-NMR: 0.94-1.08 (m, 3H), 1.10-1.14 (m, 3H), 1.16-1.20 (m, 1H), 1.25-1.29 (m, 1H), 2.11 (s, 3H), 2.47-2.58 (m, 3H), 2.90-2.99 (m, 2H), 3.12 (septet, J 6.7, 1H), 3.30 (t, J6.2, 2H), 5.55-5.59 (m, 1H), 6.94-7.20 (m, 4H).
¹³C-NMR: 14.7 (q), 15.2 (q), 20.8 (q), 33.6 (t), 38.0 (d), 39.4 (t), 39.8 (t), 42.0 (d), 42.4 (d), 114.2 (d), 123.6 (d), 124.4 (d), 126.1 (d), 126.2 (d), 134.4 (s), 136.1 (d), 141.0 (s), 149.3 (s), 168.2 (s).

### B) Tetrahydronapthalene

A solution of FeCl₃.6H₂O (1.0M in acetic acid, 1.0 ml, 1 mmol) was added to stirred solution of 1,2,3,4 tetrahydronaphthalene (21.65 g, 164 mmol), acetic anhydride (1.4 g, 13.6 mmol), acrolein diacetate (5.4 g, 34 mmol). The solution was stirred for a further 3 hours at ambient temperature, then poured into 5% sodium bicarbonate solution (200 ml), then the aqueous phase was extracted with ether (200 ml). The organic phase was washed with brine, dried over sodium sulfate, filtered and the solvents *removed in vacuo.* The residue was further purified by column chromatography on silica (300 ml) with cylohexane:ethyl acetate 19:1 and gave the enol acetates as a mixture of regio isomers (α and β (major) naphthyl, plus E and Z).
¹H NMR (both isomers): 1.70-1.86 (m, 4H), 2.11 (s, 3H), 2.64-2.80 (m, 4H), 3.21-3.29 (m, 2H), 5.55 (dt, J 12,7, 1H), 6.82-7.25 (m, 4H).
¹³C NMR(both isomers): 20.7 (q), 22.8 (t), 23.2 (t), 23.3 (t), 23.4 (t), 26.2 (t), 29.2 (t), 29.6 (t), 30.3 (t), 30.8 (t), 33.4 (t), 113.9 (d), 114.7 (d), 126.2 (d), 126.3 (d), 126.8 (d), 128.5 (d), 128.8 (d), 130.1 (d), 135.9 (s), 136.0 (s), 136.9 (d), 137.2 (d), 137.6 (s), 138.1 (s), 138.4 (d), 138.6 (d), 168.2 (s).

### C) 1,1 Dimethyl indane

A solution of FeCl₃.6H₂O (1M in acetic acid, 0.3 ml) was added slowly drop wise to a stirred solution of 1,1 dimethyl indane (4.1 g, 28 mmol) acrolein diacetate (1.1 g, 7 mmol) and acetic anhydride (0.3 g, 2.8 mmol). After 2 hours stirring at room temperature, the mixture was poured into brine (50 ml) and the aqueous phase extracted with ether (100 ml). The organic phase was washed with sodium bicarbonate (50 ml), then brine (50 ml), dried over magnesium sulfate, filtered and the solvents *removed in vacuo.* The residue was further purified by Kugelrohr distillation, 120°C at 10 mbar gave recovered 1,1 dimethyl indane (2.2 g) then distillation at 160°C at 0.3 mbar gave the enol acetates as a mixture of isomers, (1.2 g, yield: 70%).
¹H NMR: 1.25 (s, 6H), 1.91 (t, J 7, 2H), 2.11 (s, 3H), 2.84 (t, J 7, 2H), 3.32 (d, J 7, 2H), 5.58 (dt, J 12, 7, 1H), 6.95 (s, 1H), 6.96 (d, J 8, 1H), 7.10 (d, J 8, 1H), 7.18 (dt, J 12, 8, 1H).
¹³ NMR: 20.7 (q), 28.6 (q), 29.6 (q), 33.6 (t), 41.6 (t), 43.9 (t), 114.2 (d), 121.9 (d), 124.4 (d), 126.3 (d), 136.1 (d), 137.8 (s), 140.8 (s), 153.0 (s),168.1 (s)

### Example 6

### Reaction between Tert-butyl benzene and acrolein diacetate

A solution of FeCl₃.6H₂O (1M in acetic acid, 2.5 ml, 2.5 mmol) was added slowly drop wise to a stirred solution of tert-butyl benzene (55 g, 410 mmol) acrolein diacetate (13.5 g, 85 mmol) and acetic anhydride (3.5 g, 34.3 mmol). After 3 hours stirring at room temperature, the mixture was poured into brine (50 ml) and the aqueous phase extracted with ether (2 x 100 ml). The organic phase was washed with sodium bicarbonate (50 ml), then brine (50 ml), dried over magnesium sulfate, filtered and the solvents removed *in vacuo.* The residue was further purified by column chromatography on silica (500 ml) with cyclohexane then 5:95 ethyl acetate:cyclohexane gave recovered tert-butyl benzene then the enol acetates as a mixture of meta and para isomers, (2.7 g, yield: 14%).
¹H NMR: (both isomers)1.31 (s, 9H), 2.11 (s, 3H), 3.30 (d, J 8, 2H) 5.58 (dt, J 12, 8, 1H), 7.12-7.21 (m, 4H), 7.32 (d, J 8, 1H).
¹³ NMR: 20.7 (q), 30.1 (s), 31.4 (q), 33.0 (t), 34.4 (s), 112.6 (d), 113.9 (d), 125.4 (d), 127.9 (d), 128.0 (d), 134.5 (d), 136.2 (d), 136.7 (d), 136.9 (d), 149.0 (s), 149.2 (s), 168.0 (s), 168.2 (s).

### Example 7

### Reaction between Sec-butyl benzene and acrolein diacetate

A solution of FeCl₃.6H₂O (1M in acetic acid, 0.5 ml, 0.5 mmol) was added slowly drop wise to a stirred solution of *sec*-butyl benzene (11 g, 82 mmol) acrolein diacetate (2.7 g, 17 mmol) and acetic anhydride (0.7 g, 6.8 mmol) in dichloromethane (15 ml). After 3 hours stirring at room temperature, the mixture was poured into saturated sodium bicarbonate (50 ml) and the aqueous phase extracted with ether (100 ml). The organic phase was washed with saturated sodium bicarbonate (50 ml), then brine (50 ml), dried over magnesium sulfate, filtered and the solvents *removed in vacuo.* The residue was further purified by column chromatography on silica (500 ml) with cyclohexane then 5:95 ethyl acetate:cyclohexane gave recovered sec-butyl benzene then the enol acetate as a mixture of isomers, (1.3 g, yield: 33%).
¹H NMR: 0.81 (t, J 7, 3H), 1.21 (d, J 7, 3H), 1.57 (q, J 7, 2H), 2.11 (s, 3H), 2.57 (s, J7, 1H), 3.30 (dd, J 8,1,2H), 5.65-5.52 (m, 1H), 7.10-7.25 (m, 5H).
¹³ NMR: 12.2 (q), 20.7 (q), 21.8 (q), 31.2 (t), 33.19 (t), 41.3 (d), 114.0 (d), 127.2 (d), 128.2 (d), 136.2 (d), 137.0 (s), 168.2 (s).

### (Reference example)

### Example 8

### Reaction between 1,3 benzodioxole and methacrolein diacetate

Zinc chloride (0.14 g, 1 mmol, 10 mol%) was added to a stirred solution of 1,3 methylenedioxy benzene (2.4 g, 20 mmol) and methacrolein diacetate (1.72 g, 10 mmol) at ambient temperature. The solution was stirred at ambient temperature for a further 48 hours. The solution was diluted with ethyl acetate (59 ml), and sodium bicarbonate 5% (50 ml), the aqueous phase was re-extracted with ethyl acetate (50 ml), the organic phase was washed with brine (50 ml), dried over magnesium sulfate, filtered and the solvents *removed in vacuo.* The residue was further purified by column chromatography over silica (50 ml), with cylohexane then 1:19 then 1:9 ethyl acetate:cylohexane as eluant. The desired product 1.23 g was further purified by Kugelrohr distillation 125°C at 3.5 x 10⁻² mbar, to give the enol acetate, (1.0 g, yield: 53%).
¹H NMR: 1.58 (d, J 1.5, 3H), 2.14 (s, 3H), 3.17 (s, 2H), 5.91 (s, 2H), 6.63 (dd, J 8, 1.5, 1H), 6.67 (d, J 1.5, 1H), 6.72 (d, J 8, 1H), 7.02 (d, J 1.5, 1H).
¹³ NMR: 13.4 (q), 20.8 (q), 40.0 t), 100.9 (t), 108.0 (d), 109.0 (d), 121.3 (s), 121.7 (d) 131.2 (d), 132.8 (s), 146.1 (s), 147.7 (s) and 168.3 (s).

### (Reference example)

### Example 9

### Reaction between anisole and tiglic diacetate

Zinc chloride (0.14 g, 1 mmol), was added to a solution of anisole (2.16 g, 20 mmol) and tiglic diacetate (1.86 g, 10 mmol), and the mixture stirred at ambient temperature for 3 hours. The solution was diluted with ethyl acetate (25 ml) and saturated sodium bicarbonate (50 ml), the aqueous phase was re-extracted with ethyl acetate (25 ml) the combined organic phases were washed with brine, dried over magnesium sulfate, filtered and the solvents *removed in vacuo.* Further purification by column chromatography over silica (50 ml) with cyclohexane, then 1:19, then 1:9 ethyl acetate cyclohexane gave the enol acetate as a mixture of isomers. Further purification by Kugelrohr distillation 125°C at 3.5 x 10⁻² mbar gave the enol acetate as a mixture of isomers, (1.2 g, yield: 51%).
¹H NMR: 1.37 (d, J 7, 3H), 1.51 (d 1.5, 3H), 2.13 (s, 3H), 3.37 (q, J 7, 1H), 3.77 (s, 3H), 6.83 (d, J 9, 2H), 7.13 (d, J 9, 2H), 7.13 (m, 1H).
¹³ C NMR: 12.1 (q), 19.3 (q), 20.8 (q), 42.5 (d), 55.2 (q), 113.6 (d), 128.3 (d), 130.6 (d), 136.2 (s), 158.0 (s), 168.3 (s).

### Example 10

### Reaction between 2-methyl indane and crotonaldehyde diacetate

Acetic anhydride (5 g, 49 mmol) was added to a suspension of FeCl₃.6H₂O (1.08 g, 4 mmol) and 2-methyl indane (26.4 g, 200 mmol), after 5 mins crotonaldehyde diacetate (6.88 g, 40 mmol) was added slowly drop wise. The mixture was stirred for a further 7 hours, then poured into brine (50 ml), extracted with ether (100 ml), washed the organic extract with sodium bicarbonate (100 ml), then brine (50 ml), dried over magnesium sulfate, filtered and the solvents *removed in vacuo.* The residue was further purified by distillation, 65°C at 10 mbar, gave the recovered 2-methyl indane then distillation of the residue 170°C at 0.1 mbar gave the enol acetate as a mixture of isomers, (4.3 g, yield: 44%).
¹H NMR (for both major isomers): 1.11-1.17 (m, 3H), 1.34 (d, J 7, 3/2H), 1.36 (d,J 6.6, 3/2H), 2.07 (s, 3/3H), 2.09 (s, 3/3H), 2.15 (s, 3/3H), 2.45-2.58 (m, 3H), 2.96-3.08 (m, 2H), 3.45 (quintet, J 7, 1/2H), 3.96 (m, 1/2H), 5.03 (dd, J 10, 7, 1/2H), 5.61 (dd, J 12.8, 7, 1/2H), 6.94-7.20 (m, 4H).
¹³C NMR(for both major isomers): 20.7 (q), 20.9 (q), 22.0 (q), 34.5 (d), 34.8 (d), 40.8 (t), 41.1 (t), 119.3 (d), 123.0 (d), 124.4 (d), 124.8 (d), 126.0 (d), 132.6 (d), 135.0 (d), 141.9 (s), 143.5 (s), 144.2 (d), 168.7 (s).

### (Reference example)

### Example 11

### Reaction between anisole and cyclohexenyl carbaldehyde diacetate

A solution of FeCl₃.6H₂O (1M in acetic acid, 0.31 ml) was added slowly dropwise to a stirred solution of anisole (5.53 g, 51 mmol) cyclohexane carbaldehyde diacetate (2.3 g, 10.8 mmol) and acetic anhydride (0.46 g, 4.5 mmol). After 4 hours stirring at room temperature, the mixture was poured into brine (50 ml) and the aqueous phase extracted with ether (2 x 100 ml). The organic phase was washed with sodium bicarbonate (50 ml), then brine (50 ml), dried over magnesium sulfate, filtered and the solvents removed *in vacuo.* The residue was further purified by column chromatography on silica (500 ml) with cyclohexane then 5:95 ethyl acetate:cyclohexane gave the enol acetates as a mixture of isomers, (2.57 g, 91%).
MS: M(+) 260, 200, 172, 169, 121, 108, 43 m/z.

### Example 12

### Intramolecular cyclisation of 6-phenyl-hex-2-enal

A solution of FeCl₃.6H₂O (0.112M in acetic anhydride, 1.2 ml, 0.134 mmol) was added slowly dropwise to 6-phenyl-hex-2-enal (1.8 g, 10 mmol) with stirring at 5°C over 15 minutes. The reaction mixture was allowed to warm slowly to ambient temperature and stirred for a further 20 hours. The dark mixture was poured into saturated sodium bicarbonate solution, then extracted with ether (3 x 10 ml). The combined organic phase was dried over sodium sulfate, filtered and the solvents *removed in vacuo.* The residue was rapidly distilled by Kugelrohr 140-170°C at 5.0 10⁻² mbar to afford the enol acetates (2-(1,2,3,4-tetrahydro-1-naphthalenyl)vinyl acetate) as a mixture of E/Z isomers, 1.8 g, 83%.
E isomer:
   ¹H NMR: 1.68-1.70 (m, 1H), 1.71-1.80 (m, 1H), 1.86-2.01 (m, 2H), 2.12 (s, 3H), 2.72-2.84 (m, 2H), 3.41-3.49 (m, 1H), 5.49 (dd, J 13, 9, 1H), 7.05-7.19 (m, 5H).
   ¹³ NMR: 20.7 (q), 20.9 (t), 29.6 (t), 30.8 (t), 37.8 (d), 119.5 (d), 125.7 (d), 126.2 (d), 129.2 (d), 129.3 (d), 136.1 (d), 136.9 (s), 137.9 (s), 168.2 (s).
Z isomer:
   ¹H NMR: 1.54-1.64 (m, 1H), 1.73-1.83 (m, 1H), 1.89-2.03 (m, 2H), 2.18 (s, 3H), 2.75-2.86 (m, 2H), 3.99-4.06 (m, 1H), 4.98 (dd, J 10, 6, 1H), 7.05-7.14 (m, 4H), 7.16 (d, J 6, 1H).
   ¹³ NMR: 20.8 (q), 21.5 (t), 29.6 (t), 30.1 (t), 34.9 (d), 118.7 (d), 125.8 (d), 126.0 (d), 128.9 (d), 129.1 (d), 133.9 (d), 136.8 (s), 138.5 (s), 168.2 (s).

### Example 13

### Intramolecular cyclisation of 4-methyl-6-phenyl-hex-2-enal

A solution of FeCl₃.6H₂O (0.112M in acetic anhydride, 1.2 ml, 0.134 mmol) was added slowly dropwise to 4-methyl-6-phenyl-hex-2-enal (2.0 g, 10.1 mmol) with stirring at 5°C over 15 minutes. The reaction mixture was allowed to warm slowly to ambient temperature and stirred for a further 20 hours. The dark mixture was poured into saturated sodium bicarbonate solution, then extracted with ether (3 x 10 ml). The combined organic phase was dried over sodium sulfate, filtered and the solvents *removed in vacuo.* The residue was rapidly distilled by Kugelrohr 150-180°C at 5.0 10⁻² mbar to afford the enol acetates (2-(2-methyl-1,2,3,4-tetrahydro-1-naphthalenyl)vinyl acetate) as a mixture of isomers, 2:2:1:1, 1.9 g, 82%.
¹H NMR (major isomers): 0.96 (d, J 3, 3/2H), 0.98 (d, J 2.5, 3/2 H), 1.41-1.80 (m, 2H), 1.89-2.08 (m, 1H), 2.08 (s, 3/2H), 2.19 (s, 3/2H), 2.80-2.85 (m, 2H), 3.34 (dd, J 9.7, 5, 1/2H), 4.02 (dd, J 10, 5, 1/2H), 4.90 (dd, J 10.7, 6.6, 1/2H), 5.49 (dd, J 12.3, 10.2, 1/2H), 7.06-7.31 (m, 5H).
¹³C NMR(major isomers): 18.3 (q), 18.9 (q), 20.7 (q), 20.8 (q), 26.7 (t), 26.9 (t), 28.7 (t), 28.8 (t), 32.1 (d), 32.4 (d), 116.0 (d), 117.8 (d), 125.9 (d), 126.0 (d), 128.9 (d), 129.7 (d), 136.9 (s), 138.5 (s), 168.1 (d), 168.2 (d) ppm.

## Claims

1. A process for making a compound of formula 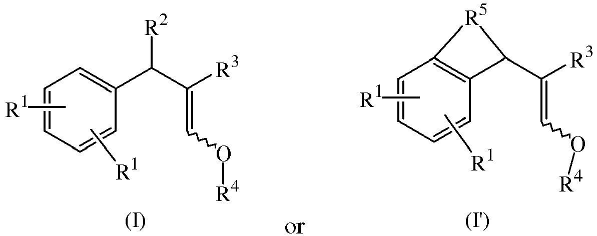
wherein the wavy line indicates that the double bond can be in a configuration E or Z or a mixture thereof;
R¹, when taken separately, one represents a hydrogen atom and the other a C₁-C₄ alkyl group; or the two R¹, when taken together, represent a C₃-C₅ alkanediyl or alkenediyl group optionally substituted;
R² or R³ represents, taken separately, a hydrogen atom or a C₁-C₆ alkyl group; R² and R³, taken together, may represent a C₃-C₁₀ alkanediyl or alkenediyl group optionally substituted;
R⁴ represents a C₁-C₇ alkyl or fluorinated alkyl group, a C₇-C₁₀ alkylaromatic optionally substituted, a C₁-C₇ acyl group, or a -COCOOH or -COCH₂COOH group; and
R⁵ represents a C₂-C₉ alkanediyl or alkenediyl group optionally substituted;
comprising the coupling of a compound of formula (II) with a compound of formula (III) 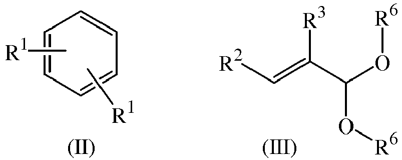
wherein R¹ to R³ have the meaning indicated in formula (I) and each R⁶, taken separately, represents a C₁-C₇ alkyl or fluorinated alkyl group, a C₇-C₁₀ alkylaromatic optionally substituted, a C₁-C₇ acyl group, or the R⁶, taken together, represent a COCO or COCH₂CO group;
or, respectively, the cyclisation of a compound of formula 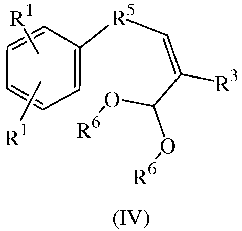
wherein R¹ and R³ have the meaning indicated in formula (I), R⁶ have the meaning indicated in formula (III), and R⁵have the meaning indicated in formula (I');
said processes being **characterized in that** it is carried out in the presence of a catalytic amount of only one catalyst selected from the group consisting of
- a salt of formula MXₙ, M representing a transition metal selected from the group consisting of Fe, Co, Ni, Cu and Zn, X representing a mono-anion and n is an integer from 1 to 3; and
- a boron compound of formula BY₃, **characterized in that** Y represents a fluoride or a phenyl group optionally substituted, and anyone of its adducts with a C₂-C₁₀ ether or a C₁-C₈ carboxylic acid.

2. A process according to claim 1, **characterized in that** the compound of formula (II) is a benzene optionally substituted by one or two C₁-C₄ alkyl groups or an indane optionally substituted by one or two C₁-C₄ alkyl groups.

3. A process according to claim 1, **characterized in that** the compound of formula (III) is acrolein diethyl acetal, acrolein diacetate, methacrolein diacetate, crotonaldehyde diacetate, tiglyl diacetate, cyclohexenyl carbaldehyde diacetate.

4. A process according to claim 1, **characterized in that** the catalyst is selected from the group consisting of BY₃ and adducts thereof, FeX₃, CoX₂, NiX₂, ZnX₂, CuX₂ and CuX.

5. A process according to claim 1, **characterized in that** the catalyst is selected from the group consisting of BY₃ and its adducts, FeX₃, and ZnX₂.

6. A process according to claim 1, **characterized in that** X is a mono-anion selected from the group consisting of acetylacetonate optionally substituted, Cl⁻, Br⁻, C₁₋₉ carboxylate, a C₁₋₁₀ sulphonate, ClO₄⁻, BF₄⁻, PF₆⁻, SbCl₆⁻, AsCl₆⁻, SbF₆⁻, AsF₆⁻ , BR⁷₄⁻ , wherein R⁷ is a phenyl group optionally substituted by one to five groups such as halide atoms or methyl or CF₃ groups, or a R⁸SO₃⁻, wherein R⁸ is a chlorine or fluoride atom.

7. A process according to claim 1, **characterized in that** X is Cl⁻, Br⁻ or trifluoromethylsulfonate.

8. A process according to claim 1, **characterized in that** the catalyst is BF₃ and its adducts with AcOH, FeCl₃, ZnBr₂ or ZnCl₂.

9. A process according to claim 1, **characterized in that** it further comprises the step of generating *in situ* the compound of formula (III) or (IV) starting from the corresponding enal of formula (V) or (V') respectively 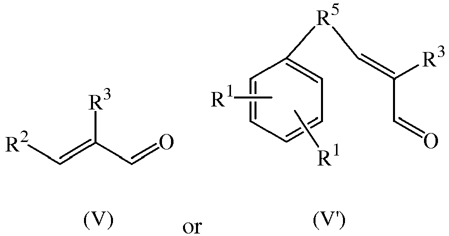 wherein R¹, R², R³ and R⁵ have the same meaning of claim 1.

## Patentansprüche

1. Verfahren zum Herstellen einer Verbindung der Formel 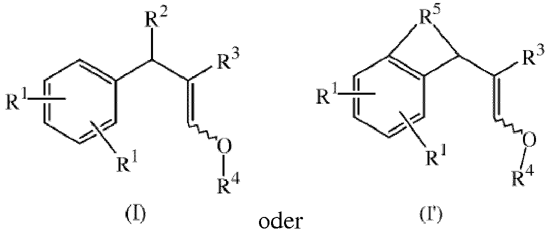
wobei die Wellenlinie anzeigt, dass die Doppelbindung in einer Konfiguration E oder Z oder einem Gemisch davon sein kann;
R¹, wenn gesondert genommen, eines ein Wasserstoffatom und das andere eine C₁-C₄-Alkylgruppe darstellt; oder die zwei R¹, wenn zusammengenommen, eine C₃-C₅-Alkandiyl- oder -Alkendiylgruppe, wahlweise substituiert, darstellen;
R² oder R³, gesondert genommen, ein Wasserstoffatom oder eine C₁-C₆-Alkylgruppe darstellt; R² und R³, zusammengenommen, eine C₃-C₁₀-Alkandiyl- oder -Alkendiylgruppe, wahlweise substituiert, darstellen können;
R⁴ eine C₁-C₇-Alkyl- oder fluorierte Alkylgruppe, eine alkylaromatische C₇-C₁₀-Gruppe, wahlweise substituiert, eine C₁-C₇-Acylgruppe, oder eine -COCOOH- oder -COCH₂COOH-Gruppe darstellt; und
R⁵ eine C₂-C₉-Alkandiyl- oder -Alkendiylgruppe, wahlweise substituiert, darstellt;
umfassend das Kuppeln einer Verbindung der Formel (II) mit einer Verbindung der Formel (III) 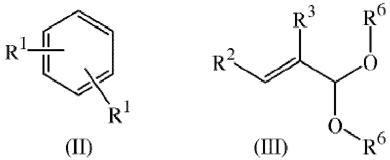
wobei R¹ bis R³ die Bedeutung, angezeigt in Formel (I), haben und jedes R⁶, gesondert genommen, eine C₁-C₇-Alkyl- oder fluorierte Alkylgruppe, eine alkylaromatische C₇-C₁₀-Gruppe, wahlweise substituiert, eine C₁-C₇-Acylgruppe darstellt, oder die R⁶, zusammengenommen, eine COCO- oder COCH₂CO-Gruppe darstellen;
oder, entsprechend, die Cyclisierung einer Verbindung der Formel 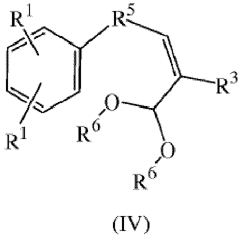
wobei R¹ und R³ die Bedeutung, angezeigt in Formel (I), haben, die R⁶ die Bedeutung, angezeigt in Formel (III), haben und R⁵ die Bedeutung, angezeigt in Formel (I'), hat; wobei die Verfahren **dadurch gekennzeichnet sind, dass** sie in Gegenwart einer katalytischen Menge von nur einem Katalysator, ausgewählt aus der Gruppe, bestehend aus
- einem Salz der Formel MXₙ, wobei M ein Übergangsmetall, ausgewählt aus der Gruppe, bestehend aus Fe, Co, Ni, Cu und Zn, darstellt, wobei X ein Monoanion darstellt und n eine ganze Zahl von 1 bis 3 ist; und
- einer Borverbindung der Formel BY₃, **dadurch gekennzeichnet, dass** Y eine Fluorid- oder eine Phenylgruppe, wahlweise substituiert, darstellt, und irgendeinem ihrer Addukte mit einem C₂-C₁₀-Ether oder einer C₁-C₈-Carbonsäure,
ausgeführt werden.

2. Verfahren gemäß Anspruch 1, **dadurch gekennzeichnet, dass** die Verbindung der Formel (II) ein Benzol, wahlweise substituiert durch eine oder zwei C₁-C₄-Alkylgruppen, oder ein Indan, wahlweise substituiert durch eine oder zwei C₁-C₄-Alkylgruppen, ist.

3. Verfahren gemäß Anspruch 1, **dadurch gekennzeichnet, dass** die Verbindung der Formel (III) Acroleindiethylacetal, Acroleindiacetat, Methacroleindiacetat, Crotonaldehyddiacetat, Tiglyldiacetat, Cyclohexenylcarbaldehyddiacetat ist.

4. Verfahren gemäß Anspruch 1, **dadurch gekennzeichnet, dass** der Katalysator aus der Gruppe, bestehend aus BY₃ und Addukten davon, FeX₃, CoX₂, NiX₂, ZnX₂, CuX₂ und CuX, ausgewählt ist.

5. Verfahren gemäß Anspruch 1, **dadurch gekennzeichnet, dass** der Katalysator aus der Gruppe, bestehend aus BY₃ und seinen Addukten, FeX₃ und ZnX₂, ausgewählt ist.

6. Verfahren gemäß Anspruch 1, **dadurch gekennzeichnet, dass** X ein Monoanion ist, ausgewählt aus der Gruppe, bestehend aus Acetylacetonat, wahlweise substituiert, Cl⁻, Br⁻, C₁₋₉-Carboxylat, einem C₁₋₁₀-Sulfonat, ClO₄⁻ , BF₄⁻ , PF₆⁻ , SbCl₆⁻ , AsCl₆⁻ , SbF₆⁻ , AsF₆⁻ , BR⁷₄⁻, wobei R⁷ eine Phenylgruppe ist, wahlweise substituiert durch eine bis fünf Gruppen, wie beispielsweise Halogenidatome oder Methyl- oder CF₃-Gruppen, oder ein R⁸SO₃⁻, wobei R⁸ ein Chlor- oder Fluoudatom ist.

7. Verfahren gemäß Anspruch 1, **dadurch gekennzeichnet, dass** X Cl⁻, Br⁻ oder Trifluormethylsulfonat ist.

8. Verfahren gemäß Anspruch 1, **dadurch gekennzeichnet, dass** der Katalysator aus BF₃ und seinen Addukten mit AcOH, FeCl₃, ZnBr₂ oder ZnCl₂ besteht.

9. Verfahren gemäß Anspruch 1, **dadurch gekennzeichnet, dass** es weiterhin den Schritt umfasst, *in situ* die Verbindung der Formel (III) oder (IV), ausgehend von dem entsprechenden Enal der Formel (V) bzw. (V') 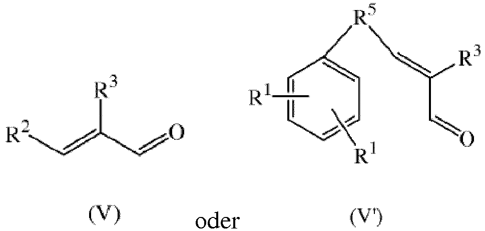 wobei R¹, R², R³ und R⁵ die gleiche Bedeutung von Anspruch 1 haben, zu generieren.

## Revendications

1. Procédé de préparation d'un composé de formule 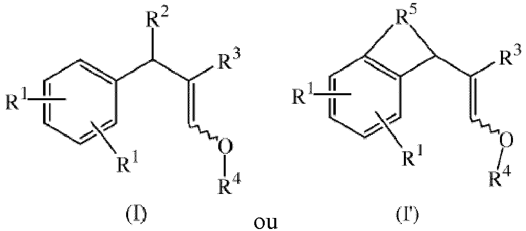
où le trait ondulé indique que la double liaison peut être dans une configuration E ou Z ou un mélange de celles-ci;
les R¹, quand ils sont pris séparément, représentent pour l'un un atome d'hydrogène et pour l'autre un groupe alkyle en C₁-C₄; ou les deux R¹, lorsqu'ils sont pris ensemble, représentent un groupe alcanediyle ou alcènediyle en C₃-C₅, éventuellement substitué; R² ou R³ représente, pris séparément, un atome d'hydrogène ou un groupe alkyle en C₁-C₆; R² et R³, pris ensemble, peuvent représenter un groupe alcanediyle ou alcènediyle en C₃-C₁₀, éventuellement substitué;
R⁴ représente un groupe alkyle ou alkyle fluoré en C₁-C₇, un alkylaromatique en C₇-C₁₀ éventuellement substitué, un groupe acyle en C₁-C₇, ou un groupe -COCOOH ou -COCH₂COOH; et
R⁵ représente un groupe alcanediyle ou alcènediyle en C₂-C₉ éventuellement substitué;
comprenant le couplage d'un composé de formule (II) avec un composé de formule (III) 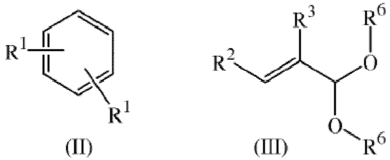
où les R¹ à R³ ont la signification indiquée dans la formule (I) et chaque R⁶, pris séparément, représente un groupe alkyle ou alkyle fluoré en C₁-C₇, un alkylaromatique en C₇-C₁₀ éventuellement substitué, un groupe acyle en C₁-C₇, ou les R⁶, pris ensemble, représentent un groupe COCO ou COCH₂CO;
ou, respectivement, la cyclisation d'un composé de formule 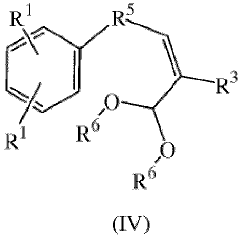
où R¹ et R³ ont la signification indiquée dans la formule (I), les R⁶ ont la signification indiquée dans la formule (III), et les R⁵ ont la signification indiquée dans la formule (I'); ledit procédé étant **caractérisé en ce qu'**il est réalisé en présence d'une quantité catalytique d'un seul catalyseur choisi dans le groupe constitué par
- un sel de formule MXₙ, M représentant un métal de transition choisi dans le groupe constitué par Fe, Co, Ni, Cu et Zn, X représentant un monoanion et n étant un entier de 1 à 3; et
- un composé de bore de formule BY₃, **caractérisé en ce que** Y représente un fluorure ou un groupe phényle éventuellement substitué, et l'un quelconque de ses produits d'addition avec un éther en C₂-C₁₀ ou un acide carboxylique en C₁-C₈.

2. Procédé selon la revendication 1, **caractérisé en ce que** le composé de formule (II) est un benzène éventuellement substitué par un ou deux groupes alkyle en C₁-C₄ ou un indane éventuellement substitué par un ou deux groupes alkyle en C₁-C₄.

3. Procédé selon la revendication 1, **caractérisé en ce que** le composé de formule (III) est du diéthylacétal d'acroléine, du diacétate d'acroléine, du diacétate de méthacroléine, du diacétate de crotonaldéhyde, du diacétate de tiglyle, du diacétate de cyclohexénylcarbaldéhyde.

4. Procédé selon la revendication 1, **caractérisé en ce que** le catalyseur est choisi dans le groupe constitué par BY₃ et ses produits d'addition, FeX₃, CoX₂, NiX₂, ZnX₂, CuX₂ et CuX.

5. Procédé selon la revendication 1, **caractérisé en ce que** le catalyseur est choisi dans le groupe constitué par BY₃ et ses produits d'addition, FeX₃, et ZnX₂.

6. Procédé selon la revendication 1, **caractérisé en ce que** X est un monoanion choisi dans le groupe constitué par un acétylacétonate éventuellement substitué, Cl⁻, Br⁻, un carboxylate en C₁₋₉, un sulfonate en C₁₋₁₀, ClO₄⁻, BF₄⁻, PF₆⁻ , SbCl₆⁻, AsCl₆⁻ , SbF₆⁻ , AsF₆⁻, BR⁷₄⁻ , où R⁷ est un groupe phényle éventuellement substitué par un à cinq groupes tels que des atomes d'halogénure ou des groupes méthyle ou CF₃, ou un R⁸SO₃⁻, où R⁸ est un atome de chlore ou de fluorure.

7. Procédé selon la revendication 1, **caractérisé en ce que** X est Cl⁻, Br⁻ ou un trifluorométhylsulfonate.

8. Procédé selon la revendication 1, **caractérisé en ce que** le catalyseur est BF₃ et ses produits d'addition avec AcOH, FeCl₃, ZnBr₂ ou ZnCl₂.

9. Procédé selon la revendication 1, **caractérisé en ce qu'**il comprend en outre l'étape de formation *in situ* du composé de formule (III) ou (IV) à partir de l'énal correspondant de formule respective (V) ou (V') 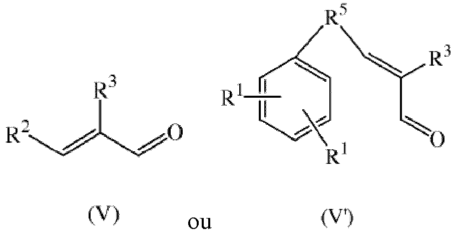 où R¹, R², R³ et R⁵ ont la même signification que dans la revendication 1.
